(19) **Europäisches Patentamt** / **European Patent Office** / **Office européen des brevets**

(11) **EP 4 489 010 B1**

(12) **EUROPEAN PATENT SPECIFICATION**

(45) Date of publication and mention
of the grant of the patent:
**02.07.2025 Bulletin 2025/27**

(21) Application number: **23306144.9**

(22) Date of filing: **06.07.2023**

(51) International Patent Classification (IPC):
***G16B 5/10*** (2019.01)

(52) Cooperative Patent Classification (CPC):
**G16B 5/10**

(54) **CALIBRATION OF A BOOLEAN NETWORK MODELING A BIOLOGICAL PROCESS**

KALIBRIERUNG EINES BOOLESCHEN NETZWERKS ZUR MODELLIERUNG EINES BIOLOGISCHEN PROZESSES

ÉTALONNAGE D'UN RÉSEAU BOOLEEN MODELISANT UN PROCESSUS BIOLOGIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**08.01.2025 Bulletin 2025/02**

(73) Proprietor: **Dassault Systèmes**
**78140 Vélizy-Villacoublay (FR)**

(72) Inventors:
• **DEMAN, Vincent**
**92260 Fontenay-aux-Roses (FR)**
• **CASTERA, Philippe**
**78140 Vélizy-Villacoublay (FR)**

(74) Representative: **Bandpay & Greuter**
**11 rue Christophe Colomb**
**75008 Paris (FR)**

(56) References cited:
• DUBROVA E ET AL: "A SAT-Based Algorithm for Finding Attractors in Synchronous Boolean Networks", IEEE/ACM TRANSACTIONS ON COMPUTATIONAL BIOLOGY AND BIOINFORMATICS, IEEE SERVICE CENTER, NEW YORK, NY, US, vol. 8, no. 5, 1 September 2011 (2011-09-01), pages 1393 - 1399, XP011412083, ISSN: 1545-5963, DOI: 10.1109/TCBB.2010.20

• FERNANDEZ DAVILA JORGE ET AL: "ZHEGALKIN POLYNOMIAL SAT SOLVER MSc. in Advanced Computer Science", 1 September 2017 (2017-09-01), XP093106939, Retrieved from the Internet <URL:https://www.academia.edu/36033079/Zhegalkin_Polynomials_SAT_Solver> [retrieved on 20231129]

• SCHWAB JULIAN D. ET AL: "Concepts in Boolean network modeling: What do they all mean?", COMPUTATIONAL AND STRUCTURAL BIOTECHNOLOGY JOURNAL, vol. 18, 1 January 2020 (2020-01-01), Sweden, pages 571 - 582, XP093107008, ISSN: 2001-0370, Retrieved from the Internet <URL:https://pdf.sciencedirectassets.com/311228/1-s2.0-S2001037019X00021/1-s2.0-S200103701930460X/main.pdf?X-Amz-Security-Token=IQoJb3JpZ2luX2VjEH0aCXVzLWVhc3QtMSJGMEQCIFZn2KRR8R5Bh5yLVpifT/HzP6mknrVebGy+b8oT3AA9AiBzy59d1cqv9M77w6I+pPYda2j2ZGnwLPWMvLmIHeHJBiq8BQjW////////8BEAUaDDA1OTAwMzU0Njg2NSIMwCmEa> DOI: 10.1016/j.csbj.2020.03.001

• CASTERA PHILIPPE: "How Living Map Can Help Research on Alzheimer's Disease We built 20 models of mechanisms leading to Alzheimer's disease with a Living Map", 14 June 2022 (2022-06-14), pages 1 - 28, XP093106997, Retrieved from the Internet <URL:https://blog.3ds.com/brands/biovia/how-living-map-can-help-research-on-alzheimers-disease/> [retrieved on 20231129]

**Description**

**TECHNICAL FIELD**

The disclosure relates to the field of computer programs and systems, and more specifically to a method, system and program for calibration of a Boolean network that models a biological process.

**BACKGROUND**

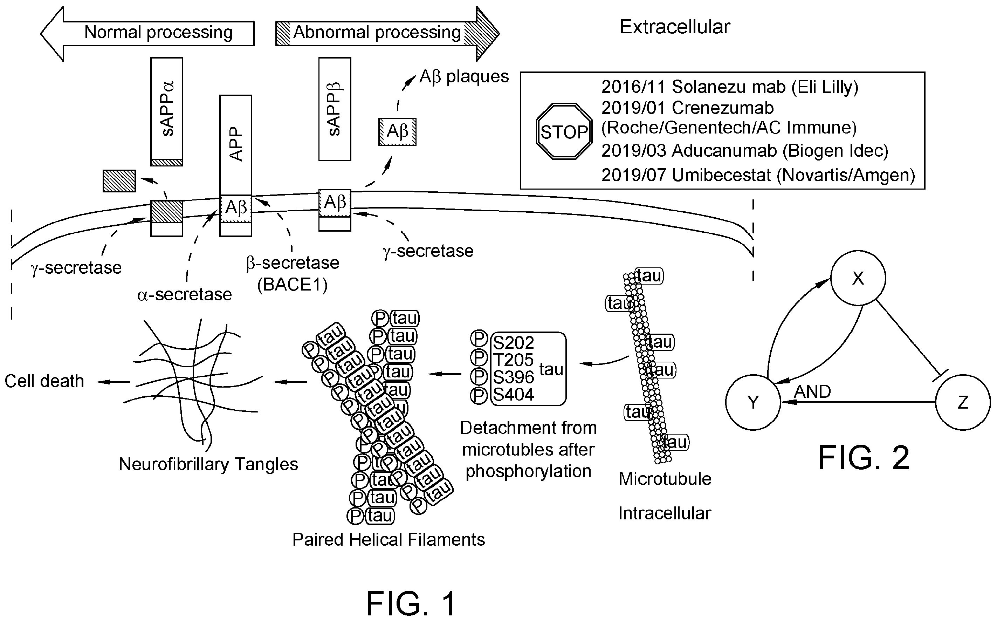

Solutions exist for modeling biological systems. Modeling a biological system has applications in the medical field, for example in the development of solutions, e.g. software or software-based solution, to assist medical practitioners in their work and/or in the development of new and/or improved drugs and/or vaccines. Example of such applications include: modeling the spread of a disease, the spread of a cancer, immuno-monitoring, inflammatory responses, effect(s) of a vaccine. The underlying biological systems, typically related to the human body and its functioning, are extremely complex. FIG. 1 shows an illustration of such system, and illustrates more specifically the two cellular processes thought to be involved in Alzheimer's Disease: formation of Amyloid plaques in the extracellular medium and accumulation of Neurofibrillary Tangles in the cell. See History and progress of hypotheses and clinical trials for Alzheimer's disease, P.-P Liu et al., Signal Transduct. Target. Ther., 4(1), 2019.

Common techniques for modeling a biological system include the use of differential systems of the type:

$$\partial tX \; = \; F(X, t, \beta).$$

However, this requires to know the function F, also referred to as "flow" or "flux", that governs the evolution of the system, and the relevant parameter(s) $\beta$. When it comes to biological systems, notably in the medical field, the knowledge of this function and of this parameter may be out of reach and/or modeling through a differential system may be not feasible. Indeed, the measurements that this knowledge would require may be out of reach.

However, biological systems, notably in the medical field, may involve regulation networks and/or influence networks, such as a sequence of activation of biological entities (e.g. genes), for example in the case of the triggering of Alzheimer's disease. FIG. 2 illustrates schematically, in a simple and schematic illustration case, such a network, where entity Y activates entity X, entity X inhibits entity Z, and entities X and Z activate entity Y.

Such networks may be modeled by Boolean networks, as well known in the art. A Boolean network is generally represented as a graph and includes: nodes (TRUE logical state, FALSE logical state), edges (Activation, Inhibition), and Logical Operators (AND ($\Lambda$), OR (v), NOT ($\neg$)). A Boolean Network (defined by function $f$) may see its state X evolve in a synchronous update scheme as:

$$X_{t+1} \; = \; f(X_t).$$

For example, the Boolean network corresponding to FIG. 2 is:

$$\begin{cases} X_{t+1} \; = \; Y_t \\ Y_{t+1} = X_t \; \wedge Z_t \; . \\ Z_{t+1} = \neg X_t \end{cases}$$

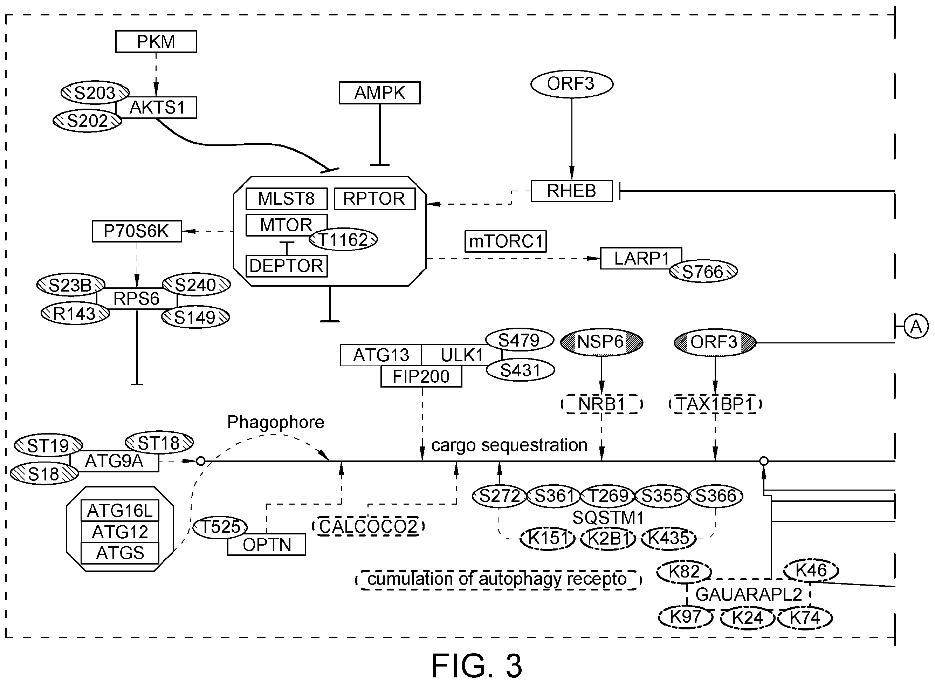

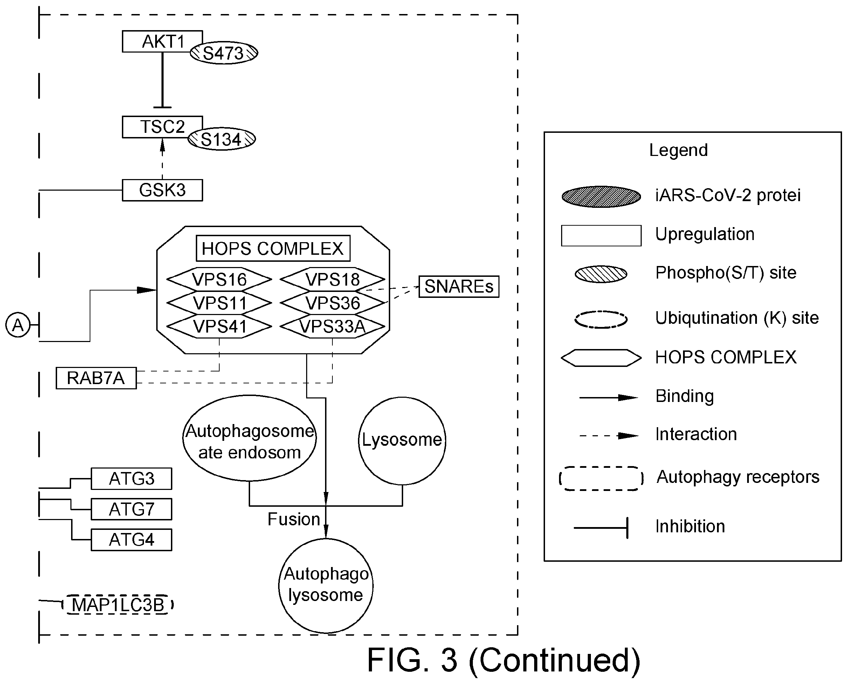

Several databases exist that store regulatory pathways as biological entities with their interactions, reactions, and relations, including: Reactome, KEGG, SIGNOR, Wikipathways, CellCollective. These entities and interactions may not fit into the Boolean formalism (such as *ubiquitination* or *phosphorylation* reactions in KEGG), but LIVING MAP (discussed below) provides a translation tool that translates these pathways into a Boolean network. FIG. 3 for example shows a screenshot of a pathway that stems from Wikipathways, that represents perturbation to host-cell autophagy induced by distinct proteins of SARS-CoV-2.

Modeling a biological system with a Boolean Network allows to perform simulations of a patient's physiology under various conditions, for example simulations of the effect of a drug or vaccine, or of the propagation of a disease or of a patient's biological response.

An online tool for Boolean Network modeling, Living Map, is provided by Dassault Systèmes, under the trademark BIOVIA. It provides for: an intuitive and userfriendly tool for biological Boolean Network modeling; interaction nodes adopting AND logic ; customization of formulas for entities; experiments under various conditions (such as initial states, gene knock-out, drugs); annotation of entities and interactions; aggregation (partly automated) of knowledge (e.g.

Uniprot data). As outlined above, Living Map also comprises a translation tool that allows the translation of regulatory pathways stored as entities and interactions (as discussed above) into Boolean Networks.

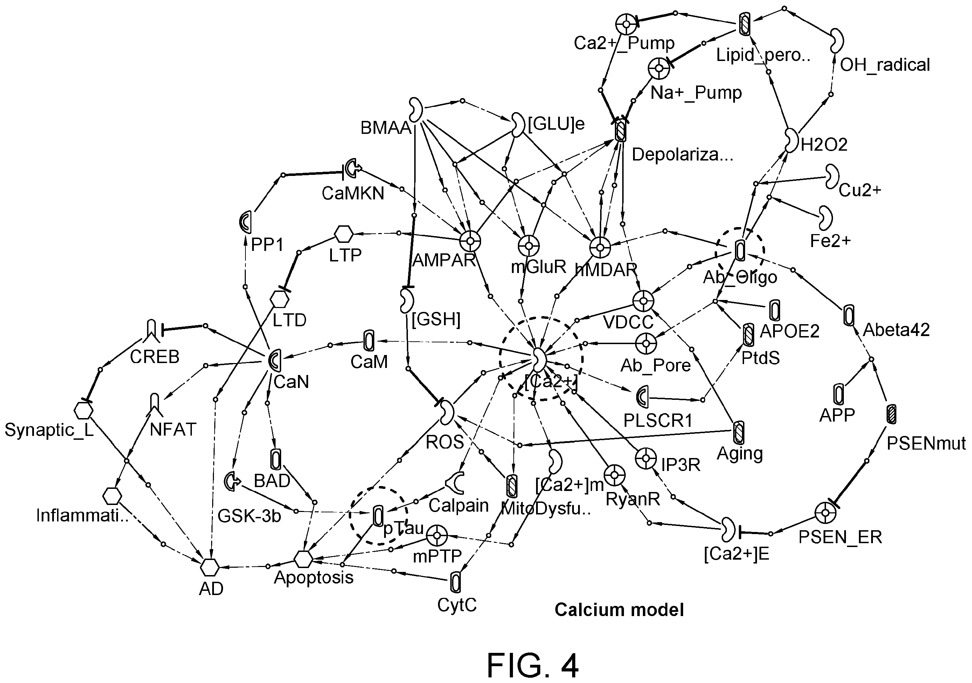

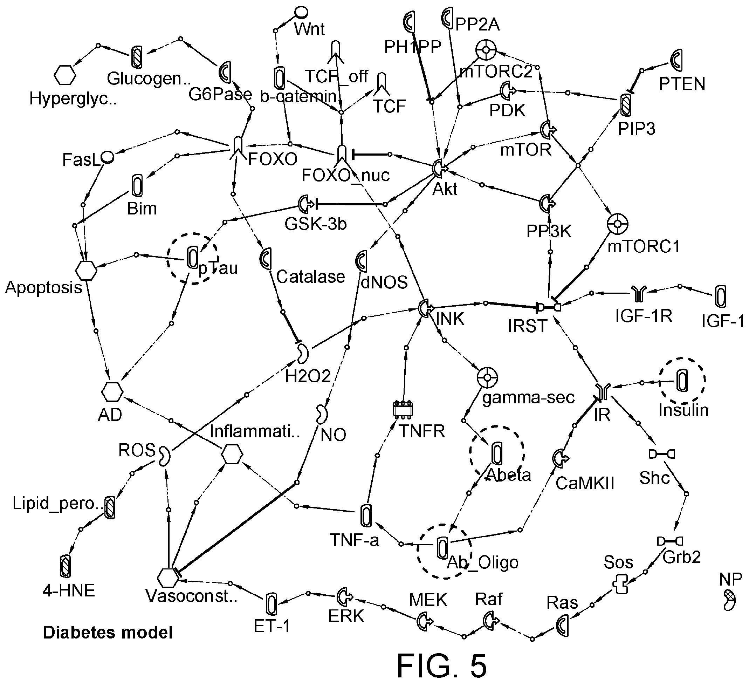

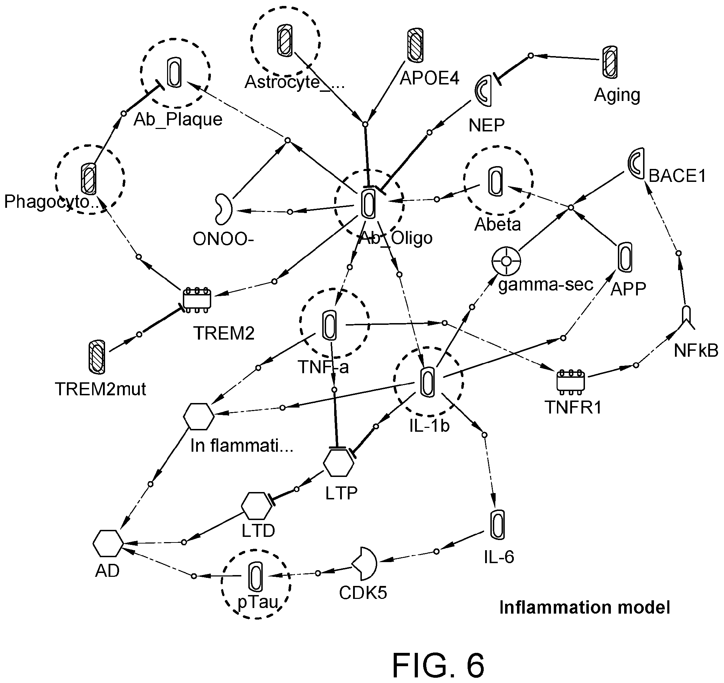

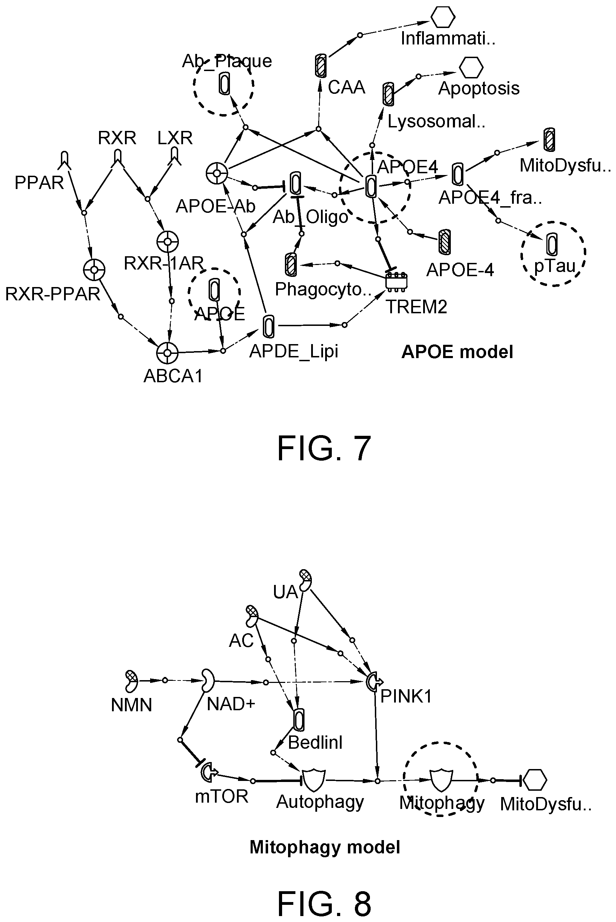

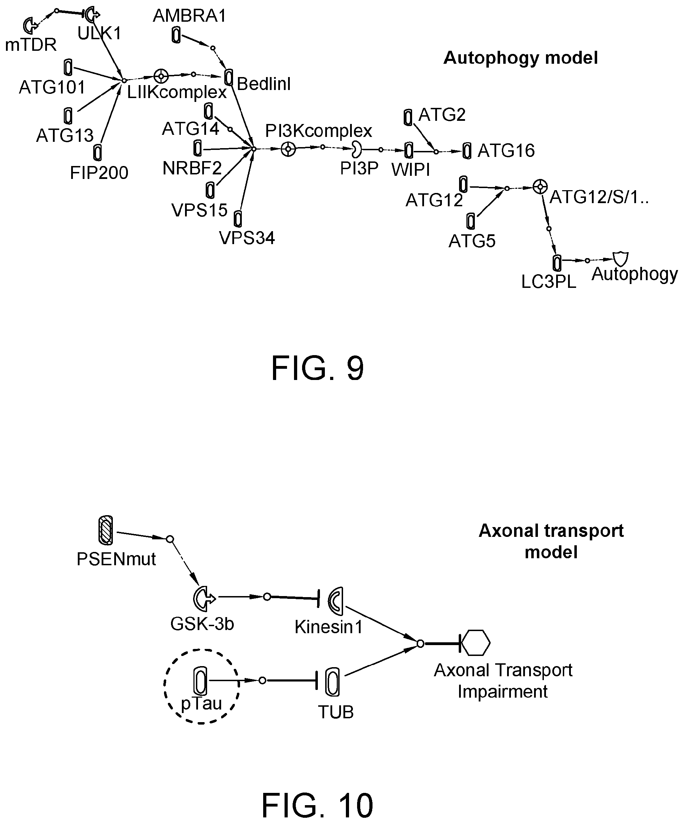

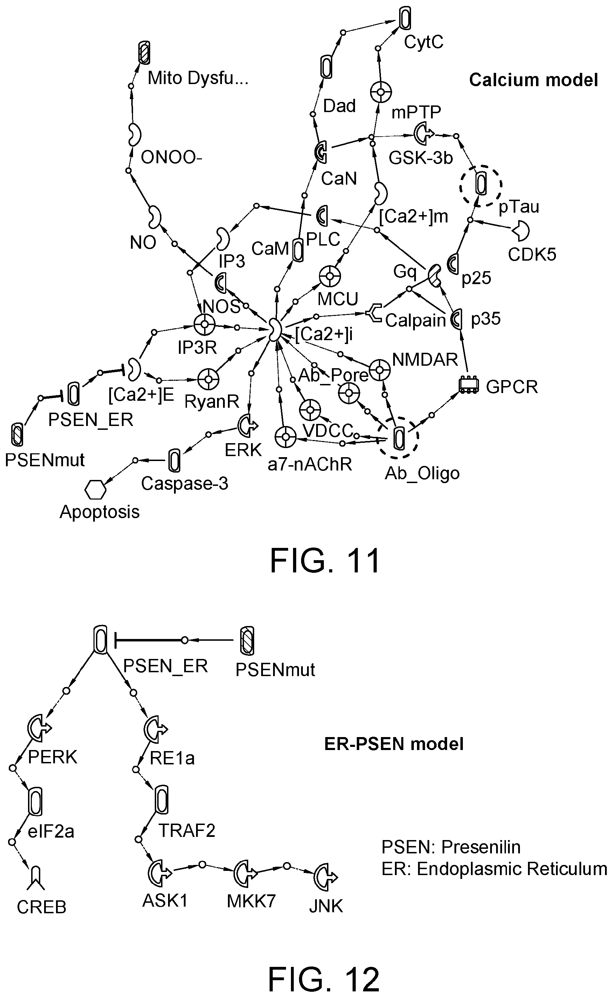

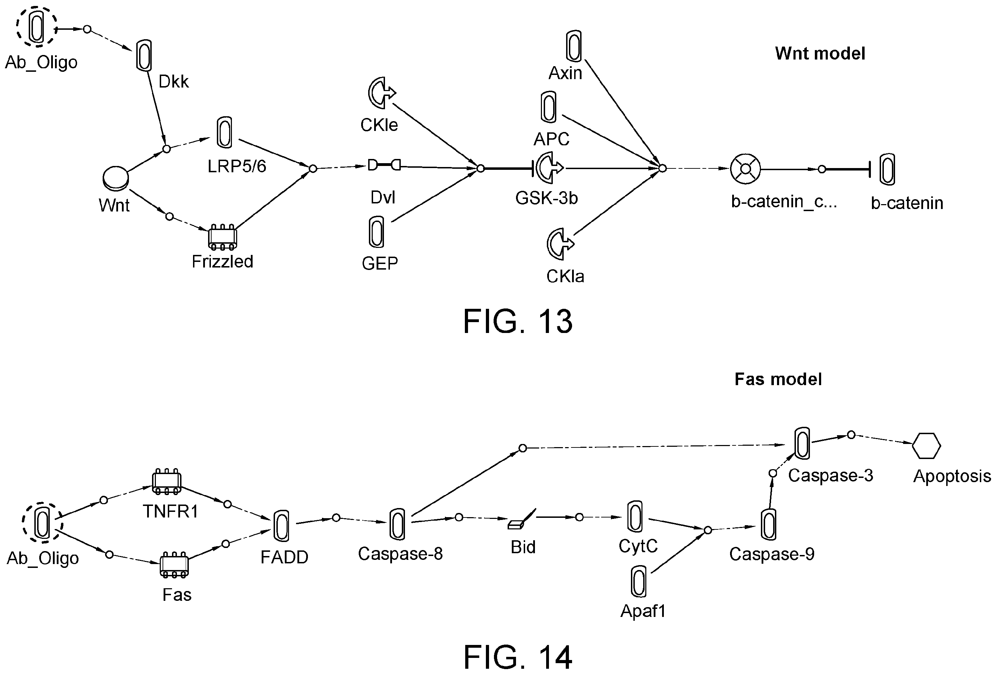

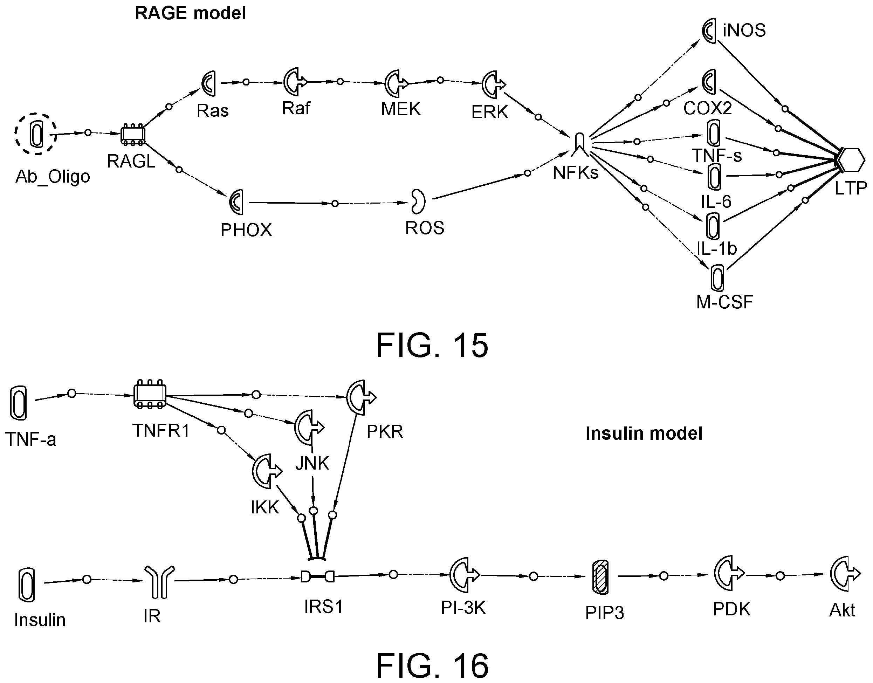

FIG.s 4 to 16 show screenshots of various models of cell signaling pathways involved in Alzheimer's disease constructed using Living Map: calcium model (FIG. 4), diabetes model (FIG. 5), inflammation model (FIG. 6), APOE model (FIG. 7), mitophagy model (FIG. 8), autophagy model (FIG. 9), axonal transport model (FIG. 10), other calcium model (FIG. 11), ER-PSEN model (FIG. 12), Wnt model (FIG. 13), Fas model (FIG. 14), RAGE model (FIG. 15), and insulin model (FIG. 16).

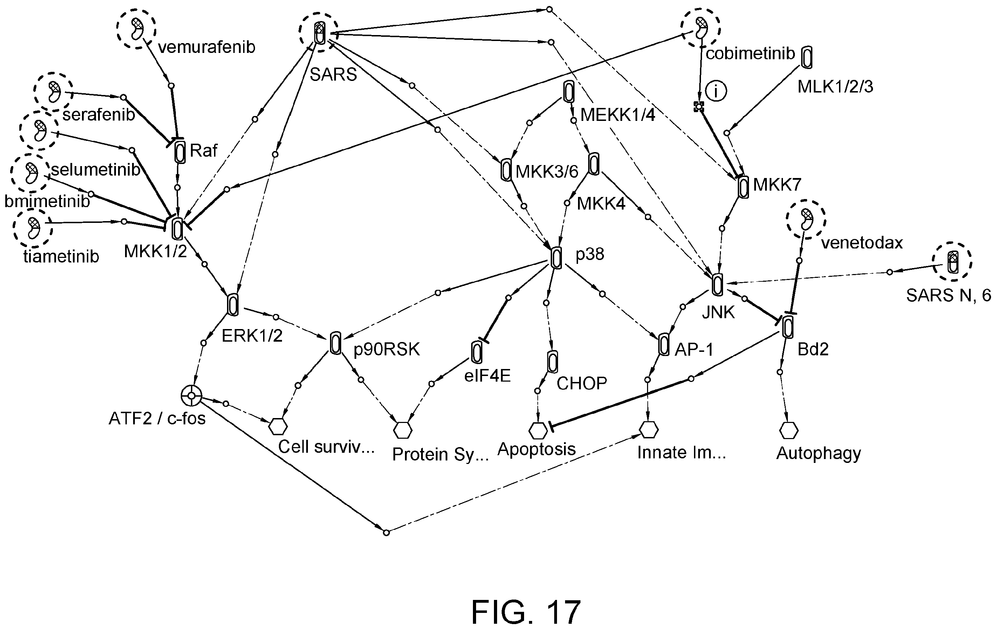

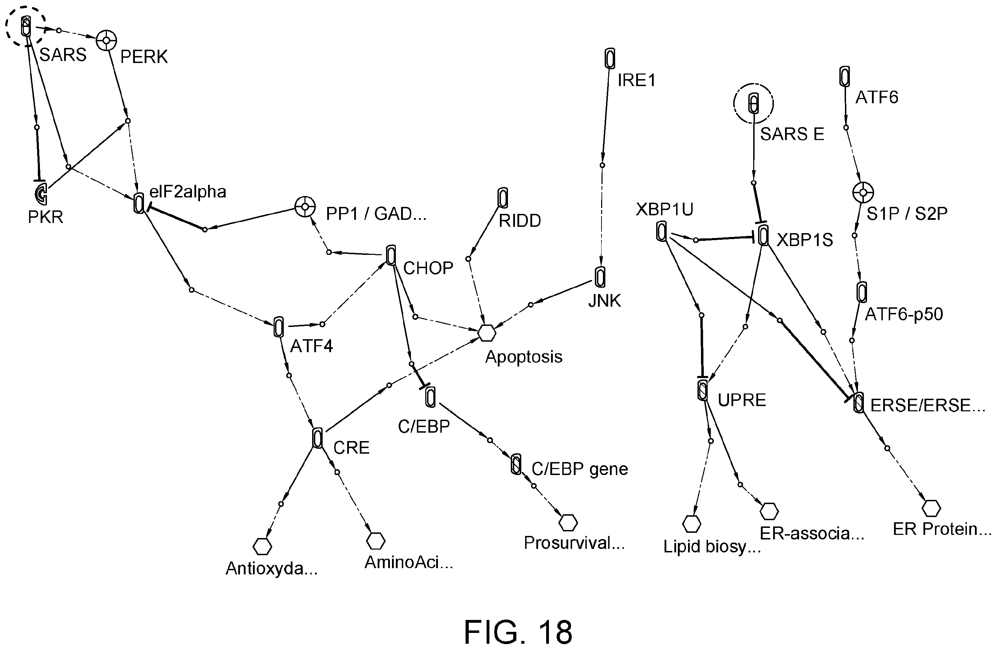

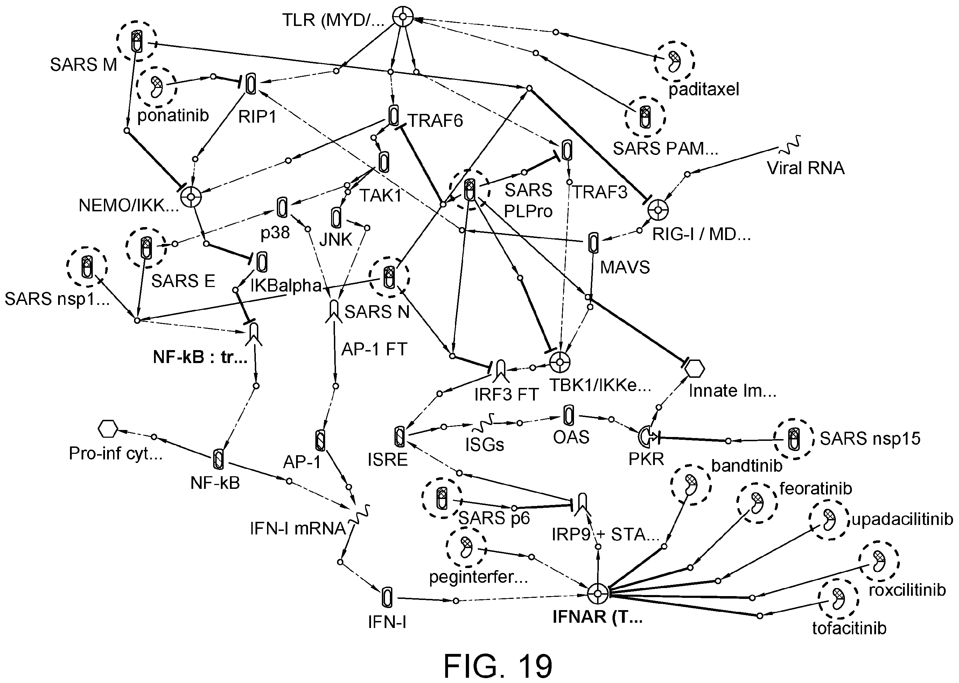

FIG. 17 shows a screenshot of a MAPK signaling model (constructed using Living Map) of the SARS-Cov-2, modeling how a coronavirus hijacks the cell machinery to stay invisible. FIG. 18 shows a screenshot of an ER stress model (constructed using Living Map) of the SARS-Cov-2, modeling how a coronavirus makes a cell build more copies of itself. FIG. 19 shows a screenshot of an innate immunity and inflammation model (constructed using Living Map) of the SARS-Cov-2, modeling how the virus blocks innate immunity and unleashes a cytokine storm.

Boolean networks thus form modern tools in the medical field for modeling the relevant biological systems, for example in the development of solutions, e.g. software or software-based solution, to assist medical practitioners in their work and/or in the development of new and/or improved drugs and/or vaccines

Within this context, there is however a need for improved solutions for calibration of Boolean networks that model biological processes. Calibration refers to the choice of Boolean functions matching a given set of data.

Also known is E. Dubrova et. al., which discloses "A SAT-Based Algorithm for Finding Attractors in Synchronous Boolean Networks", IEEE/ACM Transactions on Computational Biology and Bioinformatics, Vol. 8, No. 5, September/-October 2011, which discloses an algorithm which uses a SAT-based bounded model checking to find all attractors in a Boolean network.

## SUMMARY

The invention is defined by the appended claims.

## BRIEF DESCRIPTION OF THE DRAWINGS

Non-limiting examples will now be described in reference to the accompanying drawings, where:

- FIG.s 1 to 21 illustrate a context of the method; and

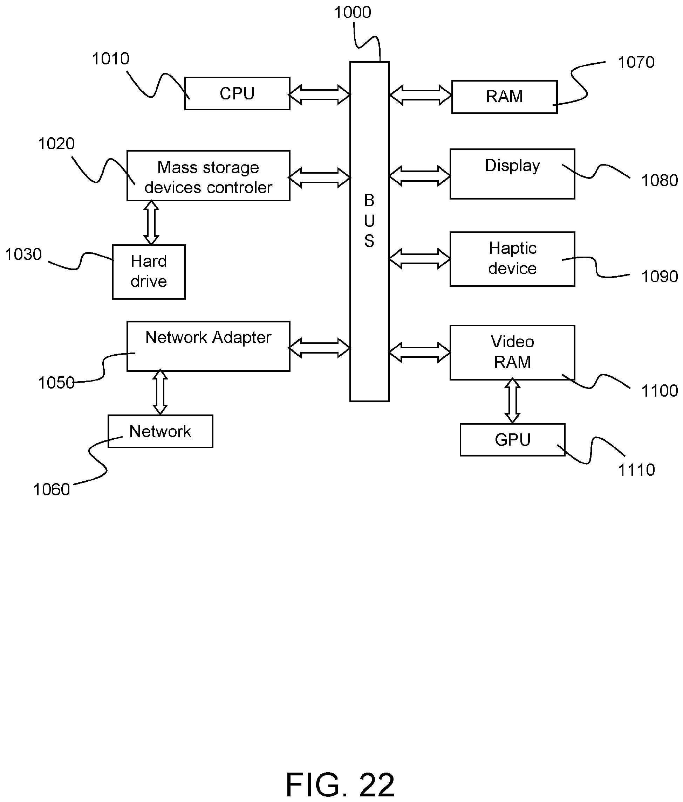

- FIG. 22 shows an example of the system.

## DETAILED DESCRIPTION

It is proposed a computer-implemented method for calibration of a Boolean network. The Boolean network has Boolean functions. The Boolean network models a biological process. The method comprises providing the Boolean network. The method further comprises providing experimental data related to the biological process. The method further comprises calibrating the Boolean network according to the experimental data. The calibration comprises converting the Boolean functions into Zhegalkin polynomials. The calibration further comprises building, based on the Zhegalkin polynomials, on the Boolean network, and on the experimental data, a calibration Boolean proposition. The calibration Boolean proposition represents a transition system of the Boolean network and the experimental data. The calibration further comprises applying a SAT solver to the calibration Boolean proposition.

This constitutes an improved solution for biological Boolean Network calibration, i.e. for calibration of a Boolean Network modeling a biological process.

Indeed, the initial (provided) Boolean network models the biological process, but at a certain level of generality. Such networks are indeed built at a certain level of generality using biological knowledge, and model the biological process at a certain level of generality, without necessarily accounting for any specific biological context, for example related to a specific patient, or of a specific medical study, or of a group of patients. The network initially provided may thus not be perfectly adapted or accurate to model the process in a specific experimental context (*e.g.* for specific physiological and/or medical data (*e.g.* measurements) of a specific patient, or of a specific medical study, or of a group of patients). A large amount of prior knowledge regarding biological processes is indeed available either directly in the literature, in dedicated databases, or even aggregated within pathways and disease maps. This knowledge may typically be used to build biological networks that mimic signaling, metabolic, or other molecular processes underlying the behavior of proteins, genes, cytokines, cells, tissues, drug mode of action, drug resistance. However, this knowledge does often not match the exact same context as the one that requires the construction of a model, and may not be exhaustive. The networks built from only that prior knowledge can overlook some mechanisms, lack specificity and only partially recapitulate experimental observations. To address that limitation, specific data from that context may be integrated. This is what the method does, by calibrating the Boolean network based on experimental data, that is specific experimental data obtained from a specific experimental context.

In other words, given the provided Boolean network, the method modifies the Boolean functions of the network while preserving its initial topology, i.e. its nodes and their interconnections (regardless of the nature of the interactions), so that its successive states reproduce as much as possible (*e.g.* up to a convergence or end criterion of the SAT solver) the successive states of the experimental data. This process is referred to as the calibration, which, as previously discussed, refers to the choice of Boolean functions matching a given set of data (here the experimental data). By performing this calibration, the method thus refines the Boolean network and makes it more fitted to specific experimental data (*e.g.* measured data). The calibrated network thereby models with particular accuracy the biological process in the specific context that is captured by the experimental data.

Now, not only does the method perform the calibration, but the method does so in a resource-efficient manner. Indeed, the main challenge of the calibration is the fact there are $2^{2^k}$ possible Boolean functions for one node depending on $k$ variables which makes their enumeration intractable in practice when $k$ increases. For example, for one node with 5 parent nodes, there are $2^{2^5} = 4,294,967,296$ Boolean functions. A brute force approach consisting in the enumeration and testing of all possible functions can therefore not be used. To solve this problem, the method first converts the Boolean functions in to Zhegalkin polynomials (also named algebraic normal form), which is a simplified polynomial representation of Boolean functions. In other words, the method converts each Boolean function into an ordinary numeric polynomial, with the Boolean *True* and *False* values replaced by 0 and 1 (the integers modulo 2), and logical conjunction (AND, $\wedge$) and exclusive disjunction (XOR, $\oplus$) as analogs of the classical product and sum. In arithmetic modulo 2, $x^2 = x$ so that the exponents become redundant, and the only nonzero coefficient is 1. Consequently, all the Boolean functions depending on $k$ variables are, once converted, fully defined by a set of $2^k$ binary coefficients. This means that, instead of searching a matching Boolean function among $2^{2^k}$ possibilities, the method reduces the search for the value (0 or 1) of those $2^k$ coefficients. In other words, the calibration performed by the method reduces the search to a combinatorial problem which is computationally feasible on available computer systems, whereas the problem of searching the matching Boolean functions directly, without the conversion into Zhegalkin polynomials, is computationally intractable on available computer systems.

Once the conversion is done, the method applies a SAT solver to the proposition that describes the transition system of the network (in which the Boolean functions, in their polynomial form, appear) and the calibration constraints extracted from the experimental data, to find the matching coefficients. A SAT solver is a computer program that solves the SAT (or Boolean satisfiability) problem, which is the problem of determining if there exists an interpretation (*i.e.* a binary value assigned to every variable) that satisfies a given Boolean formula in CNF (Conjunctive Normal Form). A formula is in CNF when it is a conjunction or disjunctive clauses, *i.e.* an AND of ORs. The use of a SAT solver in combination with the Zhegalkin conversion is thus particularly adapted and makes the method efficient. This method yields one or several interpretation(s) (i.e. a value for each one of the Zhegalkin coefficients of every function in the initial Boolean network) that is(are) compatible with the provided experimental data.

The method is for calibration of a Boolean network. The concept of Boolean network is known *per se* and has been previously discussed. A Boolean network consists of a discrete set of Boolean variables each of which has a Boolean function (possibly different for each variable) assigned to it which takes inputs from a subset of those variables and output that determines the state of the variables it is assigned to. As previously said, the Boolean network may be represented as a graph and includes: nodes (TRUE logical state, FALSE logical state), edges (Activation, Inhibition), and Logical Operators (AND ($\wedge$), OR (V), NOT ($\neg$)) and may see its state X evolve in a synchronous update scheme as (*f* representing the functions defining the network):

$$X^t = f(X^{t-1}).$$

In yet other words, the Boolean network is defined by a set of nodes representing binary variables, interconnected by a set of edges formalized as Boolean functions. By "calibration", it is meant that, given the provided Boolean network, the method modifies the Boolean functions of the network while preserving its initial topology, i.e. its nodes and their interconnection (regardless of the nature of the interactions), so that its successive states reproduce as much as possible the successive states of the experimental data. The inputs of the calibration (and of the method) are the provided Boolean network and experimental data, and the output is the Boolean network calibrated according to the experimental data.

The Boolean network models a biological process, *i.e.* any biological process. The biological process may correspond to a physiological response of a patient to a medical event. For example, the process may describe the evolution of a disease (or virus or any like medical condition) or a certain evolution phenomenon related to the disease, and/or a response of a patient to an event related to the disease (*e.g.* an inflammatory response). The model may for example be any one of the models discussed above for Alzheimer's disease or for the Coronavirus. The model may alternatively describe a response of a patient to a treatment, drug, or vaccine.

The method may for example be included in a treatment, drug, or vaccine monitoring and/or development process, for example for developing a new and/or improved treatment, drug or vaccine. The Boolean network may in this case model a physiological response to the treatment, drug or vaccine and/or a physiological process related to the disease to cure. This process may comprise one or more iterations (*e.g.* one per patient) of:

- performing the method, wherein optionally the experimental data is acquired by physical and/or physiological measurement on a patient;
- using the calibrated Boolean network to perform simulations, for example to assess the potential effects of the treatment, drug, or vaccine;
- optionally, using the results of the simulations to develop and/or improve the treatment, drug, or vaccine, for example by physically developing and/or improving the treatment, drug, or vaccine based on the results of the simulations.

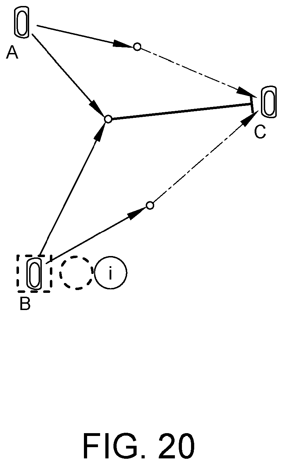

The method comprises providing (obtaining) the Boolean network. Providing the Boolean network may comprise building (*e.g.* from scratch) the network (for example using Living Map) and/or modifying an already built network (for example using Living Map). Providing the Boolean network may alternatively comprise retrieving (*e.g.* downloading) an existing Boolean network from a (*e.g.* distant) memory or server or database where the network is stored already built.
The method comprises providing (obtaining) the experimental data. Providing the experimental data may comprise retrieving (*e.g.* downloading) the experimental data from a (*e.g.* distant) memory or server or database where the data is stored. The experimental data is experimental data related to the biological process and corresponds to an experimental (*e.g.* physiological, medical) observation of the biological process (*e.g.* on a patient). The experimental data may stem from physical measurements and/or observations (*e.g.* on a patient in a medical context) of the biological process in the real world. Providing the experimental data may alternatively comprise physically acquiring the experimental data, or at least a part thereof, for example by performing physiological and/or medical measurements on a patient with suitable physical apparatuses.
Further to the providing of the inputs, the method comprises calibrating the Boolean network according to the experimental data, *i.e.* modifying the Boolean functions of the network while preserving its initial topology, i.e. its nodes and their interconnections (regardless of the nature of the interactions), so that its successive states reproduce as much as possible the successive states of the experimental data.
The calibration comprises converting the Boolean functions into Zhegalkin polynomials. Zhegalkin polynomials, also known as algebraic normal form, are representations of functions in Boolean algebra. They are the polynomial ring over the integers modulo 2. The resulting degeneracies of modular arithmetic result in Zhegalkin polynomials being simpler than ordinary polynomials, with binary coefficients and no exponents. Coefficients are binary because 1 is the only nonzero coefficient. Exponents are redundant because in arithmetic modulo 2, $x^2 = x$. Hence a polynomial such as $3x^2y^5z$ is congruent to, and can therefore be rewritten as, $xyz$. Many known methods (by using the indeterminate coefficients method, by constructing the canonical disjunctive normal form, by using tables, by using the Pascal method, by using the summation method, or by using a Karnaugh map, as discussed in https://en.wikipedia.org/wiki/Zhegalkin_polynomial) exist for converting Boolean functions into Zhegalkin polynomials, and the conversion may implement any of these methods. The method may iterate over the variables of the network and convert all the Boolean functions iteratively.
An example is now discussed to illustrate the conversion. This example considers a Boolean network with 3 variables $A, B, C$ and a Boolean formula for the variable $C = (A \lor B) \land \neg(A \lor B) = A \oplus B$. The network can be represented as illustrated on FIG. 20. The conversion may iterate over the variables. In this case only the variable C has a Boolean function that is $C = (A \lor B) \land \neg(A \lor B) = A \oplus B$. The associated Zhegalkin polynomial is $f_C(A, B) = Ж_0 \oplus Ж_1 A \oplus Ж_2 B \oplus Ж_3 AB$.
The calibration then comprises, building, based on the Zhegalkin polynomials, on the Boolean network, and on the experimental data, a calibration Boolean proposition representing a transition system of the Boolean network and the experimental data. The transition system is the equation that allows the update from $X^{t-1}$ to $X^t$. For each time point, $X^t = f(X^{t-1})$ in case one time point is equivalent to one update of the system. However, the successive states of the numerical model (here represented by $t$) may not necessarily correspond to the time reality of the experimental data (0, 3, 6, 24h for example). The scheme $X^t = f(X^{t-1}) \lor f(f(X^{t-1})) \lor ... f^N(X^{t-1})$, with $N > 1$, allows to integrate the possibility that a time difference of real time corresponds to several updates of the model. The topology of the network is fully characterized by the generic Zhegalkin polynomial of each variable and remains the same for every time point. The building of the calibration Boolean proposition may be based on the fact that $A = B$ has the same truth table as $(A \lor \neg B) \land (\neg A \lor B)$, and may comprise translating the topology condition for each time point the following proposition:

$$\left(X^t \lor \neg\left(f(X^{t-1}) \lor f(f(X^{t-1})) \lor ...\right)\right) \land \left(\neg X^t \lor \left(f(X^{t-1}) \lor f(f(X^{t-1})) \lor ...\right)\right).$$

The above proposition is referred to as the first Boolean proposition. It is comprised by the calibration Boolean proposition and represents the transition system of the Boolean network, and thereby the topology of the Boolean network. The building of the calibration Boolean proposition may also comprise, translating the experimental data into a second Boolean proposition that represents the experimental data. The second proposition may include a Boolean sub-proposition that represents a final state of the transition system according to the experimental data and/or a Boolean sub-proposition that represents an initial state of the transition system according to the experimental data and/or a Boolean sub-proposition that represents the intermediate states according to the experimental data. For example, a variable A that has the following experimental values at 4 time points: $A^0 = 0$, $A^1 = 1$, $A^2 = 1$, $A^f = 0$ is translated to the following proposition:

$$\neg A^0 \wedge A^1 \wedge A^2 \wedge \neg A^f$$

Coming back to the above example of the network illustrated on FIG. 20, if:

- The experimental data includes two time points corresponding to a single update of the network and;
- The experimental data for those two time points, with the suffix 0 designating the first/initial time point, and $f$ the second/last one, is: $A^0 = 0$, $A^f = 1$; $B^0 = 1$, $B^f = 1$; $C^0 = 0$, $C^f = 1$,

the calibration Boolean proposition includes the following first proposition for the transition system:

$$\left( C^f \vee \neg(ж_0 \oplus ж_1 A^0 \oplus ж_2 B^0 \oplus ж_3 A^0 B^0) \right)$$

$$\wedge \left( \neg C^f \vee (ж_0 \oplus ж_1 A^0 \oplus ж_2 B^0 \oplus ж_3 A^0 B^0) \right),$$

and the following second proposition for the experimental data:

$$\neg A^0 \wedge A^f \wedge B^0 \wedge B^f \wedge \neg C^0 \wedge C^f.$$

The calibration Boolean proposition may further include:

- a third Boolean proposition representing a stability constraint for the biological process, and/or
- ;
- a fourth Boolean proposition which penalizes True or False functions;
- a fifth Boolean proposition that represents a prior biological knowledge constraint

The stability constraint is any constraint that represents a stability of the transition system. The stability constraint may include at least one fixed point of the transition system and/or at least one cycle of the transition system. The concept of fixed point and of cycle is now discussed, although it is known *per se* in the field of Boolean networks. Since a Boolean network of $N$ nodes can have only $2^N$ configurations, the pigeonhole principle implies that after sufficient iterations of the update schedule, the system must return to a previously visited configuration:

$$\forall X^0, \exists(d,p) \in \mathbb{N} \times \mathbb{N}^* \mid \forall t > d, X^{t+p} = X^p;$$

$(X^{d+i})_{i \in [0,p-1]}$ is an attractor of the Boolean network;
$p$ is the attractor period; d can be considered as a distance to the attractor.

If $p = 1$, the attractor is a fixed point attractor. *If $p > 1$*, the attractor is a cycle attractor of period p. The attractor structure only depends on the network topology, no matter what initial states are considered. There must exist a cycle in the network for it to have a cyclic attractor. Some attractors have been shown to mirror many characteristics of real equilibrium states. Attractors can be a single fixed point, in that case they translate to $X^f = f(X^f)$ with $X$ the state of the system; or a cycle in which case they translate to $X^f = f^p(x^f)$ with p the period of the cycle. The third Boolean proposition may be

$$\left( X^f \vee \neg f(X^f) \right) \wedge \left( \neg X^f \vee f(X^f) \right)$$

for the fixed point case, and may be

$$\left(X^f \vee \neg(f(X^f) \vee f\left(f(X^f)\right) \vee \dots f^p(X^f))\right)$$

$$\wedge \left(\neg X^f \vee (f(X^f) \vee f\left(f(X^f)\right) \vee \dots f^p(X^f))\right)$$

for the cycle of length inferior or equal to p case.

This fourth Boolean proposition may be a conjunction of one or more disjunctive soft clauses. In the example of FIG. 20 discussed above, this fourth proposition may be

$$\neg(Ж_0 \wedge \neg Ж_1 \wedge \neg Ж_2 \wedge \neg Ж_3) \wedge \neg(\neg Ж_0 \wedge \neg Ж_1 \wedge \neg Ж_2 \wedge \neg Ж_3)$$

The effect of this fourth proposition is that the True and False single Boolean functions are to be considered for a variable only if there is no other Boolean function that matches as well or better the aforementioned constraints. The reason for that is the fact that both the True and False Boolean functions contain no mechanistic information regarding the contributions of the parent nodes of that variable.

The fifth Boolean proposition represents a prior biological knowledge constraint. The prior biological knowledge constraint may represent the Boolean network, or its functions, prior to the calibration. The prior biological knowledge constraints may be the Zhegalkin coefficients of the Boolean functions of the network prior to calibration, which carry a certain level of knowledge. The fifth Boolean proposition may thus consist in a proposition where the coefficients of the non-calibrated network's functions are true. For the aforementioned example related to FIG. 20, the initial coefficients are $Ж_0 = 0$, $Ж_1 = 1$, $Ж_2 = 1$, $Ж_3 = 0$ (that do indeed translate to $C = A \oplus B$), that is translated to:

$$\neg Ж_0 \wedge Ж_1 \wedge Ж_2 \wedge \neg Ж_3$$

For example, the resulting calibration Boolean proposition for the previous example of FIG. 20, including the stability proposition, the True/False Boolean function penalty and the prior knowledge may thus be:

$$\left(C^f \vee \neg(Ж_0 \oplus Ж_1 A^0 \oplus Ж_2 B^0 \oplus Ж_3 A^0 B^0)\right) \wedge \left(\neg C^f \vee (Ж_0 \oplus Ж_1 A^0 \oplus Ж_2 B^0 \oplus Ж_3 A^0 B^0)\right)$$

$$\wedge \neg A^0 \wedge A^f \wedge B^0 \wedge B^f \wedge \neg C^0 \wedge C^f$$

$$\wedge \left(C^f \vee \neg(Ж_0 \oplus Ж_1 A^f \oplus Ж_2 B^f \oplus Ж_3 A^f B^f)\right) \wedge \left(\neg C^f \vee (Ж_f \oplus Ж_1 A^f \oplus Ж_2 B^f \oplus Ж_3 A^f B^f)\right)$$

$$\wedge \neg(Ж_0 \wedge \neg Ж_1 \wedge \neg Ж_2 \wedge \neg Ж_3) \wedge \neg(\neg Ж_0 \wedge \neg Ж_1 \wedge \neg Ж_2 \wedge \neg Ж_3)$$

$$\wedge \neg Ж_0 \wedge Ж_1 \wedge Ж_2 \wedge \neg Ж_3$$

The calibration further comprises applying a SAT solver to the calibration Boolean proposition (in which the Boolean functions, in their polynomial form, appear) to find the matching coefficients. A SAT solver is a computer program that solves the SAT (or Boolean satisfiability) problem, which is the problem of determining if there exists an interpretation (i.e. a binary value assigned to every variable) that satisfies a given Boolean formula in CNF (Conjunctive Normal Form). A formula is in CNF when it is a conjunction or disjunctive clauses, i.e. an AND of ORs. The method may comprise converting the Boolean formulation in CNF (for example using a Tseitin transformation or the Quine-McCluskey algorithm), if necessary, prior to applying the SAT solver. The SAT solver may be a MaxSAT solver. Whereas the SAT solver aims at satisfying all the clauses (propositions), a MaxSAT solver aims at satisfying as many as possible. This may be advantageous in the case of the method. Indeed, in practice, it may happen that all the constraints may not be satisfied simultaneously, because:

- When using real experimental data, it can be subject to measurement uncertainties, missing information, inter-individual variability;
- The data is averaged and binarized: loss of information;
- The non-calibrated network fixes the topology, whereas it is not necessarily correct (often based on generic yet incomplete pathways).

The MaxSAT solver may be a weighted MaxSAT solver. In this case, each Boolean proposition included in the calibration Boolean proposition corresponds to either a conjunction of one or more disjunctive hard clauses (*i.e.* mandatory to satisfy) or a conjunction of one or more disjunctive soft clauses each having a weight. By "corresponds", it is meant the following: a Boolean proposition is herein a Boolean equation, being understood that a system of Boolean equations is also considered as a Boolean equation. A clause is a disjunctive clause, and when the Boolean proposition is fed to the MaxSAT solver (in CNF form), it is a conjunction of one or more disjunctive clauses, each being hard or soft. The method may comprise a step of translating each proposition to be fed in the MaxSAT solver in such format (CNF form). Each soft clause is given a weight and the solver aims to satisfy the clauses that maximize the sum of their weights.

The first Boolean proposition may be a conjunction of one or more disjunctive hard clauses. In other words, the transition system containing the topology of the network is fixed during calibration, and the associated constraints are marked as hard clauses (i.e. mandatory to satisfy) for each time point explicitly, to later be constrained by experimental data.

The third Boolean proposition may be a conjunction of one or more disjunctive hard clauses. Indeed, Dirichlet's box principle guarantees the system will reach a stable state within less than $2n + 1$ steps ($2n$ being the number of states of a system with n variables). In order to be able to match this stable state with experimental data, stability constraints are explicitly added as hard clauses.

The second Boolean proposition may include a sub-proposition representing the final state (attractor) of the transition system and/or a sub-proposition representing the initial state of the transition system. The sub-proposition representing the final state of the transition system may be a conjunction of one or more disjunctive soft clauses having the largest weight among all the soft clauses. In other words, priority is given to the last time point. The sub-proposition representing the initial state of the transition system may be a conjunction of one or more disjunctive soft clauses having the second largest weight among all the soft clauses. This may be advantageous, as Living Map works with a synchronous update scheme, therefore the attractor that is reached is determined by the initial state of the system. Variables without experimental data may be set to 0 by default so that the initial state is fully defined. The second Boolean proposition also includes a sub-proposition that represents the intermediary time points of the experimental data (i.e. without the initial and final states). This sub-proposition may be a conjunction of one or more disjunctive soft clauses with the third largest weight.

The fourth Boolean proposition may be a conjunction of one or more disjunctive soft clauses having the fourth largest weight among all the soft clauses.

The fifth Boolean proposition may be a conjunction of one or more disjunctive soft clauses having the smallest weight among all the soft clauses. In other words, biological information, in the shape of the non-calibrated Boolean functions (*i.e.* Zhegalkin coefficients), is only considered if all other constraints are equally satisfied.

The weights may be set by a user, for example prior to the application of the SAT solver, or may alternatively be fixed to establish a separation of the weights of the soft clauses corresponding to different Boolean propositions so that there is no overlap between these soft clauses. In this latter case each weight of a given proposition may be set to be larger than the sum of the weights of the propositions having lower weights (i.e. less important), for example equal to that sum of weights +1. The separation of the weights may be as follows:

- each weight of the sub-proposition representing the final state of the transition system is strictly larger than the sum of all the weights of the sub-proposition clauses representing the initial state of the transition system;
- each weight of the sub-proposition representing the initial state of the transition system may be strictly larger than the sum of all the weights of the sub-proposition clauses that represents the intermediary time points of the experimental data;
- each weight of sub-proposition representing the intermediary time points may be strictly larger than the sum of all the weights of the fourth proposition clauses;
- each weight of the fourth proposition may be strictly larger than the sum of all the weights of the fifth proposition clauses.

In the above, by "each weight of a proposition" or "each weight of a sub-proposition", it is meant each weight of the clauses corresponding to the proposition/sub-proposition.

This may be written as follows (and implemented as discussed above, by adding one to each of the below sums):

$$W_{final\ state} > \sum^{init\ state\ clauses} W_{init\ state}$$

$$W_{init\ state} > \sum^{intermediary\ clauses} W_{intermediary}$$

$$W_{intermediary} > \sum^{single\ Boolean\ clauses} W_{single\ Boolean}$$

$$W_{single\ Boolean} > \sum^{initial\ functions\ clauses} W_{naïve\ functions}$$

In the above, "initial functions" refers to the functions of the Boolean network as provided, that is prior to the calibration. "Single Boolean" (*i.e.* single Boolean penalty) refers to the true and false functions addressed by the fourth proposition.

This guarantees that the method will prioritize an interpretation that satisfies one more initial state clause at the expense of all intermediary state clauses, for example. Furthermore, unsatisfied clauses can be traced back to the constraints category they represent.

The Application of the SAT solver (*e.g.* weighted MaxSAT solver) then yields one or several interpretation(s) (i.e. a value for each Zhegalkin coefficient of every function in the provided Boolean network) that is(are) equally compatible with the provided calibration constraints (i.e. have the same sum of weights). The calibration may further convert back these Zhegalkin coefficients into Boolean formulae and update the Boolean network accordingly with the calibrated functions.

Five Boolean propositions included in the calibration Boolean proposition have been discussed above. It is to be understood that any other constraint or set of constraints can be added as one or more additional Boolean propositions in the calibration Boolean proposition as long as this constraint or set of constraints is translatable into a choice of values for binary variables.

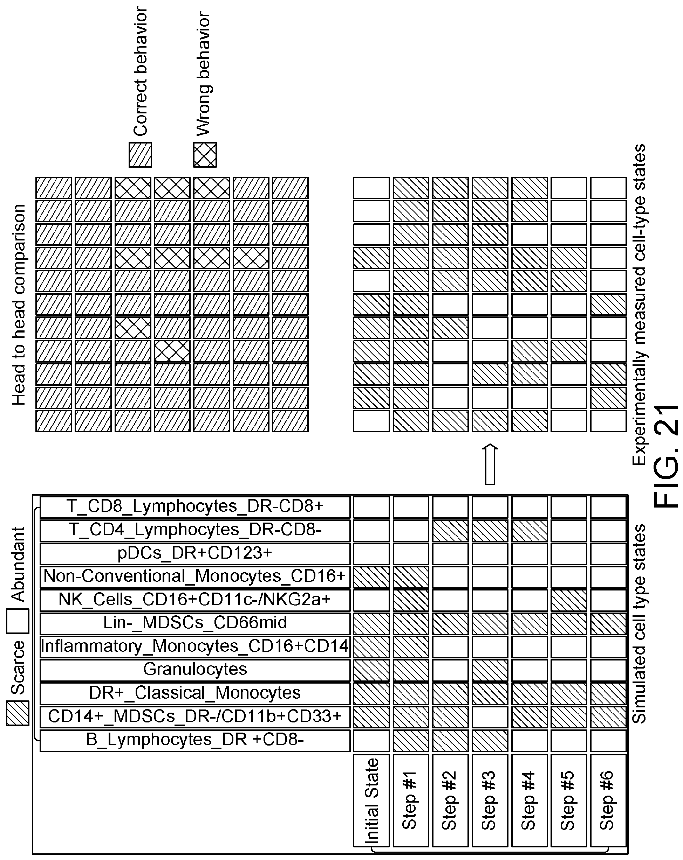

Implementations of the method have been tested by the inventors. Specifically, a biological network built to model vaccine-induced inflammation and visualize the associated cell-type abundance variations has been tested. The non-calibrated network contains 202 entities and 289 interactions. For the calibration process, the inventors used transcriptomic (for gene expressions) and cytometry (for cell abundances) data (obtained during a preclinical trial of the MVA vaccine, discussed in reference Rosenbaum P et al., Front. Immunol. 12:645210 2021 ). That data was binarized, i.e. the continuous expressions measured over the successive time points were attributed a value of 1 (meaning abundant for the cell types and expressed for the genes) or 0 (meaning scarce for the cell types and not expressed for the genes). The resulting calibrated network comprises 310 interactions and satisfies as well as possible the constraints associated with the experimental data. The results are illustrated on FIG. 21. State #6 is the final state of the model (that has reached a steady attractor). Both the initial state and final state perfectly agree with experimental data. For the transitory states, only 4 of the output makers show differences between simulated and experimental states. The calibrated functions are, however, the best matching functions with regard to the fixed parent nodes they are linked to, and their dynamics. It can thus be said that the network has been calibrated properly..

This test evidences that the method provides for an improved calibrated Boolean network, with the calibration offering the following advantages.

First, the method will output all the Boolean networks that are equally compatible with the topology given by the initial network, without needing an additional conversion or model checking step.

Also, because the method transforms the choice of the Boolean function to be assigned to each variable as an attribution of either 0 or 1 to a given number of parameters, this choice can be made with regard to any additional constraints that can also be formalized as a binary value attribution. In the case of biological network calibration, these additional constraints will always include (but are not restricted to) the binarized calibration data.

Furthermore, the SAT problem is an old and very thoroughly studied computer science problem. Several extensions of the problem have been defined, and increasingly computationally efficient ways to solve them have been developed over the years: the so-called SAT solvers, which can benefit the method. One extension in particular, the Weighted MaxSAT problem and its adequate solvers, assign a weight to each clause of the Boolean formula to be satisfied and aim at maximizing the sum of weights of the satisfied clauses, without requiring for all of them to be satisfied. This problem allows the definition of a hierarchy between clauses within a Boolean formula, i.e. in the present case, a user-controlled prioritization of the constraints associated with the calibration process and guarantees to always obtain (one of) the best matching network(s).

Finally, if no other constraints than the transition system and the data are added to the proposition to be solved, there are no assumptions made about the form of the Boolean functions, which avoids an oversimplification of the network.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 22 shows an example of the system, wherein the system is a client computer system, *e.g.* a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

**Claims**

1. A computer-implemented method for calibration of a Boolean network, the Boolean network having Boolean functions, the Boolean network modeling a biological process, the biological process being a physiological response of a patient to a treatment, drug or vaccine, the method comprising:

   - providing:

∘ the Boolean network, and
∘ experimental data related to the biological process and corresponding to an experimental observation of the biological process on the patient; and

- calibrating the Boolean network according to the experimental data, the calibration comprising:

∘ converting the Boolean functions into Zhegalkin polynomials;
∘ building, based on the Zhegalkin polynomials, on the Boolean network, and on the experimental data, a calibration Boolean proposition representing a transition system of the Boolean network and the experimental data;
∘ applying a MaxSAT solver to the calibration Boolean proposition, wherein the MaxSAT solver is a weighted MaxSAT solver, each Boolean proposition included in the calibration Boolean proposition corresponding to either a conjunction of one or more disjunctive hard clauses or a conjunction of one or more disjunctive soft clauses each having a weight, the application of the MaxSat solver modifying the Boolean functions of the Boolean network, while preserving its initial topology, so as to match the experimental data, the Boolean network being thereby adapted to model the biological process specifically for the experimental observation of the biological process on the patient, the calibrated Boolean network being thereby usable for monitoring and/or developing the drug, treatment, or vaccine,

wherein the calibration Boolean proposition includes a first Boolean proposition representing a transition system of the Boolean network and a second Boolean proposition representing the experimental data, and wherein the calibration Boolean proposition further includes:

- a third Boolean proposition representing a stability constraint for the biological process; and
- a fourth Boolean proposition which penalizes True or False functions; and
- a fifth Boolean proposition that represents a prior biological knowledge constraint,

wherein the first Boolean proposition is a conjunction of one or more disjunctive hard clauses,
wherein the third Boolean proposition is a conjunction of one or more disjunctive hard clauses,
and wherein:

- the second Boolean proposition includes a sub-proposition representing the final state of the transition system and a sub-proposition representing the initial state of the transition system, the sub-proposition representing the final state of the transition system being a conjunction of one or more disjunctive soft clauses having the largest weight among all the soft clauses, the sub-proposition representing the initial state of the transition system being a conjunction of one or more disjunctive soft clauses having the second largest weight among all the soft clauses, the second Boolean proposition also including a sub-proposition that represents the intermediary time points of the experimental data and that is a conjunction of one or more disjunctive soft clauses with the third largest weight among all the soft clauses,
- the fourth Boolean proposition is a conjunction of one or more disjunctive soft clauses having the fourth largest weight among all the soft clauses, and
- the fifth Boolean proposition is a conjunction of one or more disjunctive soft clauses having the smallest weight among all the soft clauses.

2. The method of claim 1, wherein the stability constraint includes at least one fixed point of the transition system and/or at least one cycle of the transition system, and/or the fourth Boolean proposition prevents the loss of mechanistic information, and/or the prior biological knowledge constraint represents the Boolean network prior to the calibration.

3. The method of claim 1 or 2, wherein the method comprises separating the weights of the soft clauses corresponding to different Boolean propositions so that there is no overlap between these soft clauses.

4. The method of claim 3, wherein the separation of the weights is as follows:

- each weight of the sub-proposition representing the final state of the transition system is strictly larger than the sum of all the weights of the sub-proposition clauses representing the initial state of the transition system,
- each weight of the sub-proposition representing the initial state of the transition system may be strictly larger than the sum of all the weights of the sub-proposition clauses that represents the intermediary time points of the experimental data,

- each weight of the sub-proposition that represents the intermediary time points of the experimental data may be strictly larger than the sum of all the weights of the fourth proposition clauses, and
- each weight of the fourth proposition may be strictly larger than the sum of all the weights of the fifth proposition clauses.

5. The method of any one of claims 1 to 4, wherein the experimental data stems from physical measurements and/or observations of the biological process in the realworld.

6. A computer program comprising instructions which, when executed by a computer system, cause the computer system to perform the method of any one of claims 1 to 5.

7. A computer-readable data storage medium having recorded thereon the computer program of claim 6.

8. A computer system comprising a processor coupled to a memory, the memory having recorded thereon the computer program of claim 6.

**Patentansprüche**

1. Computerimplementiertes Verfahren zur Kalibrierung eines booleschen Netzes, wobei das boolesche Netz boolesche Funktionen aufweist, das boolesche Netz einen biologischen Prozess modelliert, wobei der biologische Prozess eine physiologische Reaktion eines Patienten auf eine Behandlung, ein Arzneimittel oder einen Impfstoff ist, das Verfahren umfassend:

   - Bereitstellen:

     ◦ des booleschen Netzes, und
     ◦ experimenteller Daten, die sich auf den biologischen Prozess beziehen und einer experimentellen Beobachtung des biologischen Prozesses an dem Patienten entsprechen; und

   - Kalibrieren des booleschen Netzes gemäß den experimentellen Daten, die Kalibrierung umfassend:

     ◦ Umwandeln der booleschen Funktionen in Zhegalkin-Polynome;
     ◦ Aufbauen, basierend auf den Zhegalkin-Polynomen, des booleschen Netzes und den experimentellen Daten, eines booleschen Kalibrierungssatzes, der ein Übergangssystem des booleschen Netzes und der experimentellen Daten darstellt;
     ◦ Anwenden eines MaxSAT-Solvers auf den kalibrierten booleschen Satz, wobei der MaxSAT-Solver ein gewichteter MaxSAT-Solver ist, wobei jeder boolesche Satz, der in dem booleschen Kalibrierungssatz beinhaltet ist, entweder einer Konjunktion aus einer oder mehreren disjunktiven harten Klauseln oder einer Konjunktion aus einer oder mehreren disjunktiven weichen Klauseln entspricht, die jeweils ein Gewicht aufweisen, die Anwendung des MaxSat-Solvers die booleschen Funktionen des booleschen Netzes unter Beibehaltung seiner ursprünglichen Topologie modifiziert, um mit den experimentellen Daten übereinzustimmen, wobei das boolesche Netz angepasst ist, um den biologischen Prozess spezifisch für die experimentelle Beobachtung des biologischen Prozesses an dem Patienten zu modellieren, das kalibrierte boolesche Netz dadurch zum Überwachen und/oder Entwickeln des Arzneimittels, der Behandlung oder des Impfstoffs verwendbar ist,
     wobei der boolesche Kalibrierungssatz einen ersten booleschen Satz, der ein Übergangssystem des booleschen Netzes darstellt, und einen zweiten booleschen Satz, der die experimentellen Daten darstellt, beinhaltet, und wobei der boolesche Kalibrierungssatz ferner Folgendes beinhaltet:

       - einen dritten booleschen Satz, der eine Stabilitätsbeschränkung für den biologischen Prozess darstellt, und
       - einen vierten booleschen Satz, der die Funktionen Wahr oder Falsch bestraft, und
       - einen fünften booleschen Satz, der eine Beschränkung des biologischen Vorwissens darstellt,

         wobei der erste boolesche Satz eine Konjunktion aus einer oder mehreren disjunkten harten Klauseln ist,
         wobei der dritte boolesche Satz eine Konjunktion aus einer oder mehreren disjunktiven harten Klauseln ist,

und wobei:

- der zweite boolesche Satz einen Teilsatz, der den Endzustand des Übergangssystems darstellt, und einen Teilsatz, der den Anfangszustand des Übergangssystems darstellt, beinhaltet, der Teilsatz, der den Endzustand des Übergangssystems darstellt, eine Konjunktion aus einer oder mehreren disjunktiven weichen Klauseln mit dem größten Gewicht unter allen weichen Klauseln ist, der Teilsatz, der den Anfangszustand des Übergangssystems darstellt, eine Konjunktion aus einer oder mehreren disjunktiven weichen Klauseln mit dem zweitgrößten Gewicht unter allen weichen Klauseln ist, der zweite boolesche Satz auch einen Teilsatz beinhaltet, der die Zwischenzeitpunkte der experimentellen Daten darstellt und der eine Konjunktion von einem oder mehreren disjunktiven weichen Sätzen mit dem drittgrößten Gewicht unter allen weichen Klauseln ist,
- der vierte boolesche Satz eine Konjunktion einer oder mehrerer disjunktiver weicher Klauseln ist, die das viertgrößte Gewicht unter allen weichen Klauseln aufweisen, und
- der fünfte boolesche Satz eine Konjunktion aus einem oder mehreren disjunktiven weichen Sätzen ist, die das kleinste Gewicht unter allen weichen Klauseln aufweisen.

2. Verfahren nach Anspruch 1, wobei die Stabilitätsbeschränkung mindestens einen Fixpunkt des Übergangssystems und/oder mindestens einen Zyklus des Übergangssystems beinhaltet, und/oder der vierte boolesche Satz den Verlust mechanistischer Informationen verhindert, und/oder die Beschränkung des biologischen Vorwissens das boolesche Netz vor der Kalibrierung darstellt.

3. Verfahren nach Anspruch 1 oder 2, wobei das Verfahren ein Trennen der Gewichte der weichen Klauseln, die verschiedenen booleschen Sätzen entsprechen, umfasst, sodass es keine Überschneidung zwischen diesen weichen Klauseln gibt.

4. Verfahren nach Anspruch 3, wobei die Trennung der Gewichte wie folgt ist:

- jedes Gewicht des Teilsatzes, der den Endzustand des Übergangssystems darstellt ist streng größer als die Summe aller Gewichte der Teilsatzklauseln, die den Anfangszustand des Übergangssystems darstellen,
- jedes Gewicht des Teilsatzes, der den Anfangszustand des Übergangssystems darstellt, kann streng größer sein als die Summe aller Gewichte der Teilsatzklauseln, die die Zwischenzeitpunkte der experimentellen Daten darstellen,
- jedes Gewicht des Teilsatzes, der die Zwischenzeitpunkte der experimentellen Daten darstellt, kann streng größer sein als die Summe aller Gewichte der Klauseln des vierten Satzes, und
- jedes Gewicht des vierten Satzes kann streng größer sein als die Summe aller Gewichte der Klauseln des fünften Satzes.

5. Verfahren nach einem der Ansprüche 1 bis 4, wobei die experimentellen Daten aus physikalischen Messungen und/oder Beobachtungen des biologischen Prozesses in der realen Welt stammen.

6. Computerprogramm, umfassend Anweisungen, die, wenn sie von einem Computersystem ausgeführt werden, das Computersystem veranlassen, das Verfahren nach einem der Ansprüche 1 bis 5 durchzuführen.

7. Computerlesbares Datenspeichermedium, auf dem das Computerprogramm nach Anspruch 6 aufgezeichnet ist.

8. Computersystem, umfassend einen Prozessor, der mit einem Speicher gekoppelt ist, wobei in dem Speicher das Computerprogramm nach Anspruch 6 aufgezeichnet ist.

**Revendications**

1. Procédé mis en œuvre par ordinateur pour l'étalonnage d'un réseau booléen, le réseau booléen ayant des fonctions booléennes, le réseau booléen modélisant un processus biologique, le processus biologique étant une réponse physiologique d'un patient à un traitement, un médicament ou un vaccin, le procédé comprenant le fait de :

- fournir :

◦ le réseau booléen, et

◦ des données expérimentales relatives au processus biologique et correspondant à une observation expérimentale du processus biologique sur le patient ; et

- étalonner le réseau booléen en fonction des données expérimentales, l'étalonnage comprenant le fait de :

◦ convertir les fonctions booléennes en polynômes de Zhegalkin ;

◦ construire, sur la base des polynômes de Zhegalkin, du réseau booléen et des données expérimentales, une proposition booléenne d'étalonnage représentant un système de transition du réseau booléen et des données expérimentales ;

◦ appliquer un solveur MaxSAT à la proposition booléenne d'étalonnage, le solveur MaxSAT étant un solveur MaxSAT pondéré, chaque proposition booléenne incluse dans la proposition booléenne d'étalonnage correspondant soit à une conjonction d'une ou plusieurs clauses dures disjonctives, soit à une conjonction d'une ou plusieurs clauses souples disjonctives ayant chacune un poids, l'application du solveur MaxSAT modifiant les fonctions booléennes du réseau booléen, tout en préservant sa topologie initiale, afin de correspondre aux données expérimentales, le réseau booléen étant ainsi apte à modéliser le processus biologique spécifiquement pour l'observation expérimentale du processus biologique sur le patient, le réseau booléen étalonné étant ainsi utilisable pour le suivi et/ou la mise au point du médicament, du traitement ou du vaccin,

dans lequel la proposition booléenne d'étalonnage comprend une première proposition booléenne représentant un système de transition du réseau booléen et une deuxième proposition booléenne représentant les données expérimentales, et dans lequel la proposition booléenne d'étalonnage comprend en outre :

- une troisième proposition booléenne représentant une contrainte de stabilité pour le processus biologique ; et
- une quatrième proposition booléenne qui pénalise les fonctions Vrai ou Faux ; et
- une cinquième proposition booléenne qui représente une contrainte de connaissances biologiques préalables, dans lequel la première proposition booléenne est une conjonction d'une ou plusieurs clauses dures disjonctives, dans lequel la troisième proposition booléenne est une conjonction d'une ou plusieurs clauses dures disjonctives, et dans lequel :

- la deuxième proposition booléenne comprend une sous-proposition représentant l'état final du système de transition et une sous-proposition représentant l'état initial du système de transition, la sous-proposition représentant l'état final du système de transition étant une conjonction d'une ou plusieurs clauses souples disjonctives ayant le poids le plus fort parmi toutes les clauses souples, la sous-proposition représentant l'état initial du système de transition étant une conjonction d'une ou plusieurs clauses souples disjonctives ayant le deuxième poids le plus fort parmi toutes les clauses souples, la deuxième proposition booléenne comprenant également une sous-proposition qui représente les points temporels intermédiaires des données expérimentales et qui est une conjonction d'une ou plusieurs clauses souples disjonctives ayant le troisième poids le plus fort parmi toutes les clauses souples,
- la quatrième proposition booléenne est une conjonction d'une ou plusieurs clauses souples disjonctives ayant le quatrième poids le plus fort parmi toutes les clauses souples, et
- la cinquième proposition booléenne est une conjonction d'une ou plusieurs clauses souples disjonctives ayant le poids le plus faible parmi toutes les clauses souples.

2. Procédé selon la revendication 1, dans lequel la contrainte de stabilité inclut au moins un point fixe du système de transition et/ou au moins un cycle du système de transition, et/ou la quatrième proposition booléenne empêche la perte d'informations mécanistiques, et/ou la contrainte de connaissances biologiques préalables représente le réseau booléen avant l'étalonnage.

3. Procédé selon la revendication 1 ou 2, dans lequel le procédé comprend le fait de séparer les poids des clauses souples correspondant à des propositions booléennes différentes de sorte qu'il n'y a pas de chevauchement entre ces clauses souples.

4. Procédé selon la revendication 3, dans lequel la séparation des poids est comme suit :

- chaque poids de la sous-proposition représentant l'état final du système de transition est strictement supérieur à la somme de tous les poids des clauses de sous-proposition représentant l'état initial du système de transition,

- chaque poids de la sous-proposition représentant l'état initial du système de transition peut être strictement supérieur à la somme de tous les poids des clauses de sous-proposition représentant les points temporels intermédiaires des données expérimentales,

- chaque poids de la sous-proposition qui représente les points temporels intermédiaires des données expérimentales peut être strictement supérieur à la somme de tous les poids des clauses de la quatrième proposition, et

- chaque poids de la quatrième proposition peut être strictement supérieur à la somme de tous les poids des clauses de la cinquième proposition.

5. Procédé selon l'une quelconque des revendications 1 à 4, dans lequel les données expérimentales proviennent de mesures physiques et/ou d'observations du processus biologique dans le monde réel.

6. Programme informatique comprenant des instructions qui, lorsqu'elles sont exécutées par un système informatique, amènent le système informatique à mettre en œuvre le procédé selon l'une quelconque des revendications 1 à 5.

7. Support de stockage de données lisible par ordinateur sur lequel est enregistré le programme informatique selon la revendication 6.

8. Système informatique comprenant un processeur couplé à une mémoire, sur laquelle mémoire est enregistré le programme informatique selon la revendication 6.

FIG. 1

FIG. 2

FIG. 3

FIG. 3 (Continued)

Calcium model

FIG. 4

EP 4 489 010 B1

FIG. 5

Inflammation model

FIG. 6

**FIG. 7**

**FIG. 8**

Autophogy model

FIG. 9

Axonal transport
model

FIG. 10

23

FIG. 11

**ER-PSEN model**

PSEN: Presenilin
ER: Endoplasmic Reticulum

FIG. 12

FIG. 13

FIG. 14

EP 4 489 010 B1

**RAGE model**

FIG. 15

**Insulin model**

FIG. 16

FIG. 17

FIG. 18

FIG. 19

FIG. 20

FIG. 21

FIG. 22

**REFERENCES CITED IN THE DESCRIPTION**

**Non-patent literature cited in the description**

- **P.-P LIU et al.** History and progress of hypotheses and clinical trials for Alzheimer's disease. *Signal Transduct. Target. Ther.*, 2019, vol. 4 (1)

- **E. DUBROVA**. A SAT-Based Algorithm for Finding Attractors in Synchronous Boolean Networks. *IEEE/ACM Transactions on Computational Biology and Bioinformatics*, September 2011, vol. 8 (5)
- **ROSENBAUM P et al.** *Front. Immunol.*, 2021, vol. 12, 645210